# EUROPÄISCHE PATENTANMELDUNG

(11) **EP 2 444 117 A1**
(43) Veröffentlichungstag der Anmeldung: **25.04.2012**
(21) Anmeldenummer: 10188134.0
(22) Anmeldetag: 20.10.2010
(51) Int. Cl.: A61M 39/20

(54) **Schutzkappe für einen Konnektor**

(71) Anmelder: Fresenius Kabi Deutschland GmbH, 61352 Bad Homburg (DE)
(72) Erfinder: Rahimy, Ismael, 61169, Friedberg (DE); Brandenburger, Torsten, 61203, Reichelsheim (DE)
(74) Vertreter: Richly, Erik

(57) **Zusammenfassung**

Die Erfindung betrifft eine Schutzkappe zum Verschließen eines Konnektors für medizinischen Wirkstoff enthaltende Behälter, ein Konnektorsystem und ein Verfahren zum Reinigen und/oder Desinfizieren einer Oberfläche einer zurückgesetzten Membran eines Konnektors.

Die erfindungsgemäße Schutzkappe (1, 1') umfasst einen äußeren Kappenbereich (2) und einen inneren Kappenbereich (3), wobei äußerer Kappenbereich (2) und innerer Kappenbereich (3) zwischen sich einen im Wesentlichen ringförmigen Hohlraum (4) für den Eingriff eines ringförmigen Anschlussstücks (21) eines Konnektors (20) ausbilden, wobei der innere Kappenbereich (2) einen Tupfer (5, 5', 5") aufweist zum Reinigen und/oder Desinfizieren einer zum Rand (22) des Anschlussstücks (21) zurückgesetzten Membran (23) des Konnektors (20).

## Beschreibung

### Technisches Gebiet

Die Erfindung betrifft eine Schutzkappe zum Verschließen eines Konnektors für medizinischen Wirkstoff oder Nährlösung enthaltende Behälter, ein Konnektorsystem und ein Verfahren zum Reinigen und/oder Desinfizieren einer Oberfläche einer zurückgesetzten Membran eines Konnektors mit einer Schutzkappe.

### Stand der Technik

Die Druckschrift US7763006B2 offenbart eine Schutzkappe, die auf einen spritzenförmigen Portreiniger aufsetzbar ist. Im Inneren der Schutzkappe ist am Boden der Schutzkappe ein ringförmiger Schwamm vorgesehen, der Desinfektionsmittel aufweisen kann.

Die Druckschrift WO2009123709 A2 offenbart eine Reinigungsvorrichtung, die ein kappenförmiges Gehäuse und einen im Gehäuse angeordneten Schwamm aufweist. Der Schwamm ist mit Desinfektionsmitteln getränkt. Des Weiteren umfasst die Reinigungsvorrichtung einen Deckel, der das Gehäuse mit dem Schwamm verschließt um den Schwamm im sterilen Zustand zu halten.

Die Druckschriften WO2010/034470A1 und WO2005/037362A1 offenbaren Konnektoren für medizinischen Wirkstoff enthaltende Behälter, beispielsweise für Infusionsbeutel- oder Transfusionsbeutel.

### Kurzbeschreibung der Erfindung

Die erfindungsgemäße Schutzkappe zum Verschließen eines Konnektors für medizinischen Wirkstoff enthaltende Behälter umfasst einen äußeren Kappenbereich und einen inneren Kappenbereich, wobei äußerer Kappenbereich und innerer Kappenbereich zwischen sich einen im Wesentlichen ringförmigen Hohlraum für den Eingriff eines ringförmigen Anschlussstücks eines Konnektors ausbilden, wobei der innere Kappenbereich einen Tupfer aufweist zum Reinigen und/oder Desinfizieren einer zum Rand des Anschlussstücks zurückgesetzten Membran des Konnektors.

Der Begriff "ringförmig" soll insbesondere auch im Wesentlichen konische, zylindrische oder gemischte Formen umfassen.

Bei der Verwendung von Behältern, die Konnektoren mit einer zurückgesetzten Membran aufweisen, beispielsweise Konnektoren wie in den Schriften WO2010/034470A1 und WO2005/037362A1 beschrieben, besteht die Möglichkeit, dass die Oberfläche der Membran, beispielsweise durch Herausziehen einer zuvor in den Konnektor eingeführten Spritze, Nadel oder eines Spikes, durch den Inhalt des Behälters, beispielsweise ein flüssiges Arzneimittel oder eine Nährstofflösung, benetzt wird. Bei giftigen Inhalten des Behälters stellt eine benetzte Oberfläche der Membran ein Risiko für die Benutzer dar, beispielsweise für das Krankenhauspersonal. Des Weiteren kann durch die Benetzung der Oberfläche der Membran eine Keimbildung begünstigt sein, was eine weitere Entnahme von Inhalt aus dem Behälter nach einem gewissen Zeitablauf einschränkt oder ganz unmöglich macht.

Durch die erfindungsgemäße Schutzkappe ist es möglich, Konnektoren mit zurückgesetzter Membran effektiv zu reinigen und/oder zu desinfizieren. Gleichsam ermöglicht die Schutzkappe ein sicheres Verschließen der Öffnung des Konnektors. Die Schutzkappe lässt sich einfach auf den Konnektor aufsetzen, wobei durch die Ausbildung des inneren Kappenbereiches mit Tupfer gewährleistet ist, dass der Tupfer die zurückgesetzte Membran sicher erreichen und reinigen und/oder desinfizieren kann.

Vorzugsweise ist der Tupfer derart ausgebildet, dass die gesamte, zum Außenbereich hin frei liegende Oberfläche gereinigt und/oder desinfiziert werden kann. Bevorzugt kann der Tupfer derart ausgebildet sein, dass er zusätzlich die an die Membran angrenzende Innenfläche des Anschlussstücks mitreinigt und/oder desinfiziert. Hierzu kann der Tupfer beispielsweise ein scheibenförmiges, der Membran entgegengerichtetes Ende mit geeigneter Dicke aufweisen.

Vorzugsweise umfasst der innere Kappenbereich einen Haltevorsprung, an dem der Tupfer befestigt ist. Ein solcher Haltevorsprung ermöglicht eine sichere und dauerhafte Befestigung des Tupfers.

Vorzugsweise ist der innere Kappenbereich mit einer Außenfläche ausgebildet, die zur Innenfläche des Anschlussstückes des Konnektors zumindest bereichsweise korrespondiert, so dass mit dem Aufsetzen des Konnektors diese beiden Flächen zumindest in einem Abschnitt umlaufend aufeinander zu liegen kommen. Auf diese Weise ist es möglich, eine Dichtung zwischen Schutzkappe und Konnektor zu erzeugen, die das Risiko eines unerwünschten Auslaufens eines Inhalts aus dem Behälter verringern kann.

Besonders bevorzugt korrespondiert die Außenfläche des Haltevorsprungs mit der Innenfläche des Anschlussstückes, so dass mit dem Aufsetzen des Konnektors diese beiden Flächen zumindest in einem Abschnitt umlaufend aufeinander zu liegen kommen. Ist die Schutzkappe aus einem steifen Material ausgebildet, was bevorzugt ist, kann auf diese Weise mit einem ebenfalls aus einem steifen Material bestehenden Anschlussstück eine einfach herzustellende und zu lösende Abdichtung erreicht werden.

Besonders bevorzugt ist, dass die Schutzkappe als Male-Teil eines Luer-Konnektors oder eines Luer-Lock-Konnektors ausgebildet ist (ISO 594-1:1986 / EN 20594-1 :1993 bzw. ISO 594-2:1998 / EN1707:1996).

Vorzugsweise weist der Tupfer ein Reinigungsmittel, beispielsweise ein Sterilisationsmittel oder Desinfektionsmittel, auf. Der Tupfer kann beispielsweise flüssigkeitsaufsaugend ausgebildet sein und mit einem derartigen Reinigungsmittel getränkt sein.

Weitere vorteilhafte Ausführungsformen der Erfindung sind Gegenstände der abhängigen Ansprüche.

Die Erfindung wird im Folgenden anhand von Ausführungsbeispielen, die durch mehrere Figuren dargestellt sind, näher erläutert.

### Kurze Beschreibung der Zeichnungsfiguren

Es zeigt:

Fig.1 eine perspektivische Ansicht einer ersten Ausführungsform einer erfindungsgemäßen Schutzkappe,

Fig. 2 eine Ansicht der in Fig. 1 gezeigt Schutzkappe, bei der der Tupfer weggelassen wurde.

Fig. 3 eine Ansicht der in Fig. 1 gezeigt Schutzkappe mit Tupfer,

Fig. 4 einen Schnitt durch die Schutzkappe mit einem ersten Tupfer,

Fig. 5 einen Schnitt durch die Schutzkappe mit einem zweiten Tupfer,

Fig. 6 einen Schnitt durch die Schutzkappe mit einem dritten Tupfer,

Fig. 7 einen Schnitt durch die Schutzkappe mit dem zweiten Tupfer und einer Sperrfolie,

Fig. 8 eine perspektivische Ansicht eines Konnektorsystem mit einer Schutzkappe gemäß erster Ausführungsform und einem Konnektor,

Fig. 9 einen Längsschnitt durch das in Fig. 8 gezeigte Konnektorsystem ,

Fig. 10 eine perspektivische Ansicht einer zweiten Ausführungsform einer Schutzkappe,

Fig. 11 einer weitere perspektivische Ansicht der in Fig. 10 gezeigten Schutzkappe,

Fig. 12 eine perspektivische Ansicht eines Konnektorsystem mit einer Schutzkappe gemäß zweiter Ausführungsform und einem Konnektor, und

Fig. 13 einen Längsschnitt durch das in Fig. 12 gezeigte Konnektorsystem.

### Beschreibung der Ausführungsarten

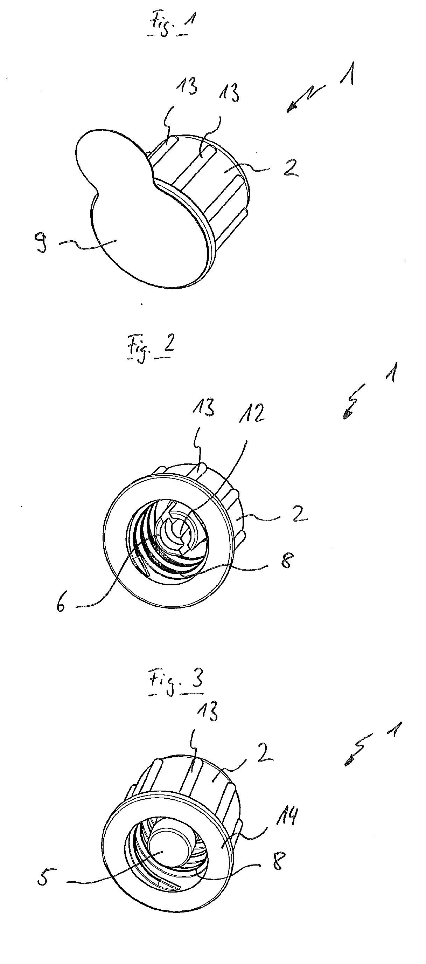

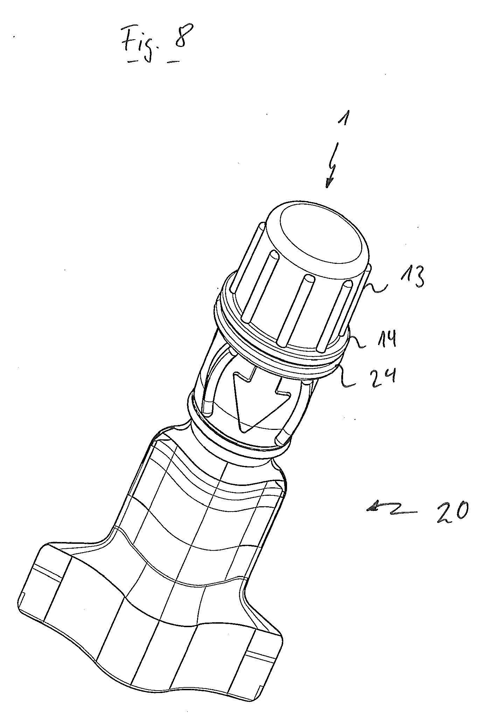

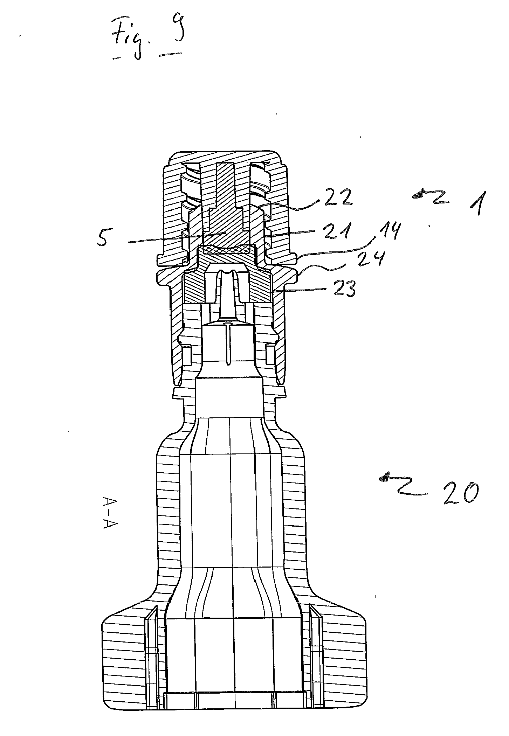

Die Fig. 1 bis 7 zeigen in verschiedenen Ansichten eine erste Ausführungsform einer erfindungsgemäßen Schutzkappe 1. Die Fig. 8 und 9 zeigen ein Konnektorsystem, das eine derartige Schutzkappe 1 und einen Konnektor 20 aufweist.

Die Schutzkappe 1 ist zum sicheren Verschließen eines Konnektors 20 für medizinischen Wirkstoff oder Nährlösung enthaltende Behälter, beispielsweise von Beuteln oder Flaschen, wie Sie in der Infusion, Transfusion, klinischen Ernährung oder Dialyse verwendet werden, ausgebildet. Die Schutzkappe umfasst einen äußeren Kappenbereich 2, einen inneren Kappenbereich 3 und einen Kappenboden 7. Der äußere Kappenbereich 2 und der innere Kappenbereich 3 sind über den Kappenboden 7 miteinander verbunden. Der äußere Kappenbereich 2 und der innere Kappenbereich 3 bilden zwischen sich, d.h., zwischen der Innenfläche des äußeren Kappenbereich 2 und der Außenfläche des inneren Kappenbereich 3, einen im Wesentlichen ringförmigen Hohlraum 4 für den Eingriff eines ringförmigen Anschlussstücks 21 des Konnektors 20 aus. Die Außenfläche des äußeren Kappenbereichs weist mehrere Rippen 13 auf, die eine Handhabung der Schutzkappe 1 erleichtern.

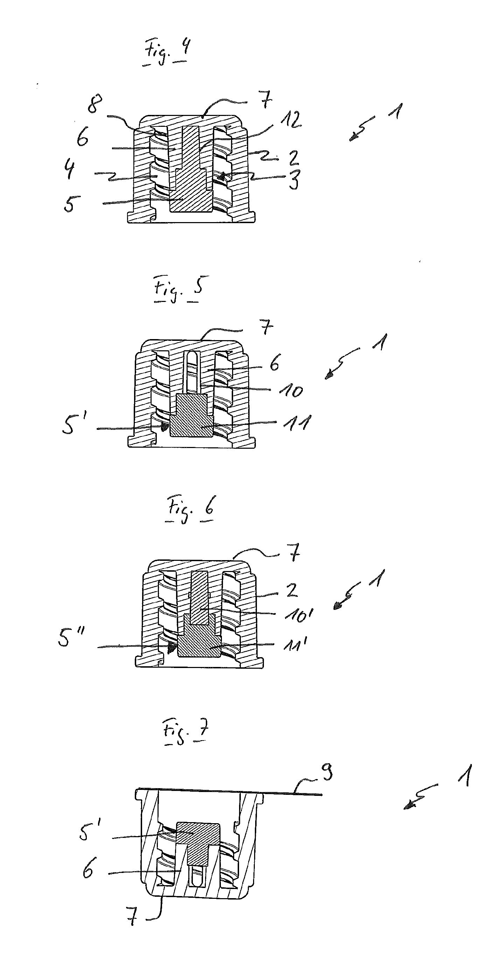

Der innere Kappenbereich 3 umfasst einen Haltervorsprung 6 und einen Tupfer 5. Der Haltevorsprung 6 ist im Zentrum des Kappenbodens 7 angeordnet. Der Außendurchmesser des Haltevorsprung 6 weist hier die Maße eines Luer-Konus auf. Der Haltevorsprung 6 umfasst eine Aussparung 12, in die der Tupfer 5 eingesetzt ist. Die Aussparung 12 ermöglicht eine sichere Befestigung des Tupfers 5 an dem Haltevorsprung 6. In diesem Ausführungsbeispiel ist die Aussparung 12 in Längsrichtung stufenförmig ausgebildet, siehe beispielsweise Fig. 4-7.

Äußerer Kappenbereich 2, Kappenboden 7 und Haltevorsprung 6 sind als einstückiges Spritzgussteil aus einem harten Plastik, beispielsweise aus PP, PE, PA, PS oder Mischung aus PP und SEBS, hergestellt. Dadurch ist die Schutzkappe 1 kostengünstig herstellbar. Andere Materialien oder Materialmischungen oder ein mehrteiliger Aufbau sind aber grundsätzlich ebenfalls möglich.

Der Tupfer 5 ist zum Reinigen und/oder Desinfizieren einer zum Rand 22 des Anschlussstücks 21 zurückgesetzten Membran 23 des Konnektors 20 ausgebildet, siehe Fig. 9. Der Tupfer 5 erstreckt sich über den Haltervorsprung 6 des inneren Kappenbereichs 3 hinaus. Dies gewährleistet, dass der Tupfer 5 die Membran 23 möglichst großflächig, vorzugsweise die gesamte freiliegende Oberfläche, berühren, reinigen und/oder desinfizieren kann. In diesem Ausführungsbeispiel ist der Tupfer 5 mit seinem der Membran 23 zugewandten Endabschnitt scheibenförmig ausgebildet.

Die Fig. 4-6 zeigen verschiedene Varianten des Tupfers.

In einer ersten Variante, siehe Fig. 4, ist der Tupfer 5 einteilig ausgebildet. Der Tupfer 5 wird vorzugsweise durch Füllen des Haltevorsprungs 6 mit einem flüssigkeitssaugenden Material hergestellt, beispielsweise aus Polyurethanen, ABS, Nylon, Polyester oder aus Polyolefinen.

In einer zweiten Variante, siehe Fig. 5, ist der Tupfer 5' zweiteilig mit einem Halteteil 10 und einem Tupferteil 11 ausgebildet. Das Tupferteil 11 besteht vorzugsweise aus Kunst-, Schaumstoff, Papier oder Baumwolle, das über einen Hinterschnitt in den Hohlraum des Halteteils 10, im Haltevorsprung 6 durch Verpressen, Verkleben oder Verklemmen fixiert ist

In einer weiteren Variante, siehe Fig. 6, ist der Tupfer 5" ebenfalls zweiteilig mit einem Halteteil 10 und einem Tupferteil 11 ausgebildet. In diesem Falle ist das Halteteil 10 als Stäbchen ausgebildet, dass über einen Formschluss im Haltevorsprung 6 befestigt ist.

Der einteilige Tupfer 5 bzw. das Tupferteil 11 sind vorzugsweise aus einem weichen, besonders bevorzugt aus einem elastischem, Material, um sich an die Oberfläche der Membran 23 anpassen zu können und diese vollflächig berühren zu können.

Der Tupfer 5, 5', 5" ist in diesen Ausführungsbeispielen mit einem Desinfektionsmittel getränkt, beispielsweise mit Isopropanol, Ethanol, Polyvidon-Jod oder einem anderen Desinfektionsmittel. Andere Reinigungsmittel sind grundsätzlich ebenfalls möglich.

Der äußere Kappenbereich 2 weist ein Befestigungsmittel 8 auf, dass es ermöglicht, die Schutzkappe 1 dauerhaft mit einem Konnektor 20 zu verbinden. In dieser Ausführungsform ist das Befestigungsmittel 8 als Innengewinde ausgebildet, dass mit einem Außengewinde des Konnektors 20 (nicht sichtbar) zusammenwirken kann. Aufgrund dessen ist die Schutzkappe 1 dauerhaft, aber lösbar, an dem Konnektor 20 befestigbar.

Die Schutzkappe 1 umfasst des Weiteren eine Sperrfolie 9. Die Sperrfolie 9 verschließt luftdicht den Tupfer 5, 5', 5" umfassenden Innenbereich der Schutzkappe 1. Damit verhindert die Sperrfolie 9 eine Kontam ination des Innenbereichs und auch die Verdunstung des Reinigungsmittels. Die Sperrfolie 9 ist in diesem Ausführungsbeispiel umlaufend an dem Außenrand 14 des äußeren Kappenbereichs 2 befestigt, beispielsweise mit diesem verschweißt oder verklebt. Die Sperrfolie 9 ist mit einer überstehenden Lasche ausgebildet, die ein einfaches manuelles Ablösen der Sperrfolie 9 ermöglicht. Die Sperrfolie 9 kann beispielsweise eine Aluminiumverbundmfolie oder eine Kunststoffmono- oder Verbundfolie sein.

Die Außenfläche des inneren Kappenbereichs 3 ist hier konisch ausgebildet. Insbesondere ist die Schutzkappe 1 als Male-Teil eines Luer-Lock-Konnektors ausgebildet.

Die Fig. 8 und 9 zeigen eine Ausführungsform eines erfindungsgemäßen Konnektorsystem, das eine Schutzkappe 1 und ein Konnektor 20 umfasst. Derartige Konnektoren 20 sind beispielsweise aus der WO2010/034470 A1 und WO 2005/037362 A1 bekannt. Soweit es um den Aufbau solcher Konnektoren 20 geht wird auf die genannten Druckschriften verwiesen.

In diesem Ausführungsbeispiel ist der Konnektor 20 im Bereich des Anschlussstücks 21 als Female-Teil eines Luer-Konnektors ausgebildet. Durch das Aufsetzen der Schutzkappe 1 auf das Anschlussstück 21 wird, neben der Reinigung und/oder Desinfizierung der Oberfläche der zurückgesetzten Membran 23 durch die vollflächige Berührung der Membran 23 durch den Tupfer 5, auch eine Dichtung hergestellt, dadurch, dass ein umlaufender Abschnitt der konischen Außenfläche des Haltevorsprungs 6 an der konischen Innenfläche des Anschlussstücks 21 umlaufend zu liegen kommt. Die Schutzkappe 1 verhindert auf diese Weise nicht nur eine Kontamination der Membranoberfläche, sondern bietet auch einen Auslaufschutz.

Dadurch, dass der Tupfer 5 scheibenförmig über den Haltervorsprung 6 hinaussteht, wird auch eine Reinigung und/oder Desinfektion des an die Membran 23 angrenzenden Abschnitts der Innenfläche des Anschlussstücks 21 erreicht.

Der innere Kappenbereich 3 der Schutzkappe 1 ist mit einer derartigen Länge ausgebildet, dass die Oberfläche der Membran zwar 23 berührt, aber die Membran 23 nicht geöffnet wird. Damit bleibt eine Dichtwirkung der Membran 23, beispielsweise im Falle einer selbstabdichtenden Membran 23, erhalten. Um die Bewegung der Schutzkappe 1 bis zur dieser Endstellung zu begrenzen, weist der Konnektor 20 angrenzend an das Anschlussstück 21 einen kragenförmigen Randbereich 24 auf. Der Randbereich 24 bildet einen Anschlag für den Rand 14 des äußeren Kappenbereichs 2. Auf diese Weise ist eine Endstellung der Schutzkappe 1 definiert.

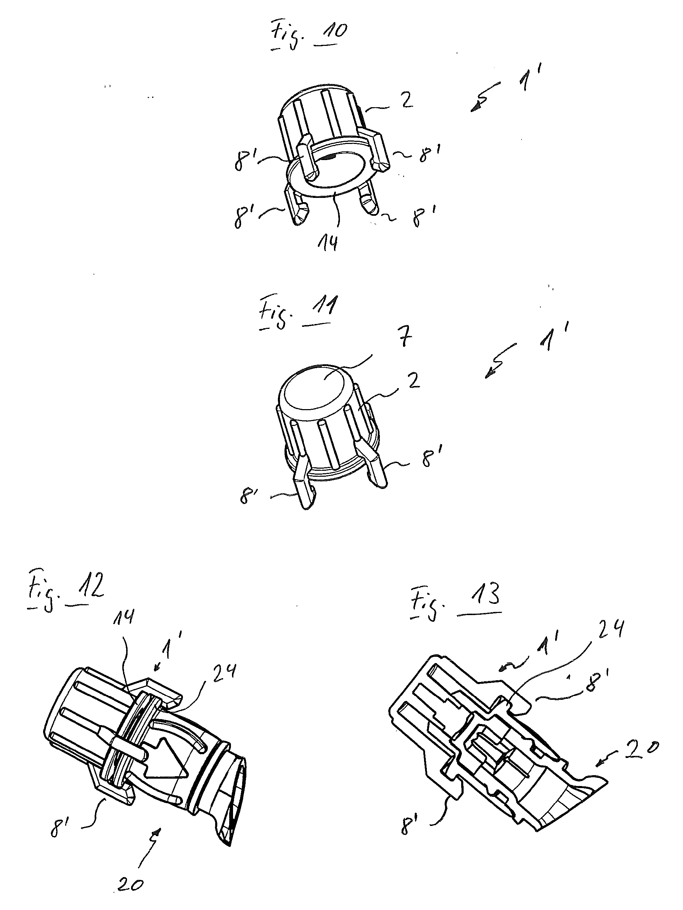

Die Fig. 10-13 zeigen eine zweite Ausführungsform einer erfindungsgemäßen Schutzkappe 1' und eines erfindungsgemäßen Konnektorsystems in verschiedenen Ansichten.

In Abweichung zur ersten Ausführungsform der Schutzkappe 1 ist die Schutzkappe 1' mit Befestigungsmitteln 8' ausgebildet, die eine dauerhafte, nicht lösbare Befestigung der Schutzkappe 1 an dem Konnektor 20 ermöglichen. Die Befestigungsmittel 8' sind hier als vier federnd nachgebende Haken ausgebildet, die umlaufend an der Außenfläche des äußeren Kappenbereichs 2 angeformt sind. Beim Aufsetzen der Schutzkappe 1 auf den Konnektor 20 und Erreichen der Endstellung haken sich die Haken 8' federnd hinter den Randbereich 24 des Konnektors 20 ein. Ein Abziehen der Schutzkappe 1 von dem Konnektor 20 ist nicht mehr möglich. Dadurch wird eine erneute Benutzung des Konnektors 20 verhindert, gleichsam wird die Membran 23 gereinigt und/oder desinfiziert und eine Kontamination des Behälterinhalts des mit dem Konnektor 20 verbundenen Behälters verhindert.

Die Haken 8' können mit dem äußeren Kappenbereich 2, inneren Kappenbereich 3 und Kappenboden 7 einstückig oder mehrteilig als Spritzgussteil aus Plastik ausgebildet sein.

Als alternative Variante kann die Schutzkappe 1' mit den Schnapphaken auch lösbar gestaltet sein. Bei dieser Variante kann auf das Innengewinde der Kappe verzichtet werden. Das Befestigen der Kappe mit dem Konnektor erfolgt über Schnapphaken, die hier nicht zur dauerhaften Befestigung, sondern zur lösbaren Befestigung ausgebildet sind. Beispielsweise kann die Schutzkappe in der Form ausgebildet sein, dass die Haken bei einem ausreichend hohen Zug auf die Schutzkappe nachgeben und sich aus ihrer Stellung, in der Sie den Randbereich 24 des Konnektors 20 hintergreifen, selbständig lösen (beispielsweise in dem die Haken anstatt eines rechtwinkligen mit einem schrägen oder abgerundeten Hintergriff ausgebildet sind).

## Patentansprüche

1. Schutzkappe (1 , 1') zum Verschließen eines Konnektors (20) für medizinischen Wirkstoff enthaltende Behälter, umfassend einen äußeren Kappenbereich (2) und einen inneren Kappenbereich (3), wobei äußerer Kappenbereich (2) und innerer Kappenbereich (3) zwischen sich einen im Wesentlichen ringförmigen Hohlraum (4) für den Eingriff eines ringförmigen Anschlussstücks (21) eines Konnektors (20) ausbilden, wobei der innere Kappenbereich (3) einen Tupfer (5, 5', 5") aufweist zum Reinigen und/oder Desinfizieren einer zum Rand (22) des Anschlussstücks (21) zurückgesetzten Membran (23) des Konnektors (20).

2. Schutzkappe (1, 1') nach Anspruch 1, wobei der innere Kappenbereich (3) einen Haltevorsprung (6) aufweist, an dem der Tupfer (5, 5', 5") befestigt ist.

3. Schutzkappe (1, 1') nach Anspruch 2, wobei der Tupfer (5, 5', 5") sich über den Haltevorsprung (6) hinauserstreckt.

4. Schutzkappe (1 , 1') nach einem der vorhergehenden Ansprüche, zusätzlich umfassend einen Kappenboden (7), wobei der äußere Kappenbereich (2) und der innere Kappenbereich (3) über den Kappenboden (7) miteinander verbunden sind.

5. Schutzkappe (1 , 1') nach einem der vorhergehenden Ansprüche, wobei der innere Kappenbereich (3) eine konische Außenform aufweist.

6. Schutzkappe (1 , 1') nach einem der vorhergehenden Ansprüche, wobei der Tupfer (5, 5', 5") einteilig ausgebildet ist und vorzugsweise Kunststoff, Schaumstoff, Papier oder Baumwolle umfasst, oder zumindest zweiteilig aufgebaut ist mit einem Halteteil (10, 10'), das fest mit dem Haltevorsprung (6) verbunden ist, und einem Tupferteil (11, 11'), das fest mit dem Halteteil (10, 10') verbunden ist, wobei das Tupferteil (11, 11') vorzugsweise aus Kunststoff, Schaumstoff, Papier oder Baumwolle umfasst.

7. Schutzkappe (1 , 1') nach einem der vorhergehenden Ansprüche, wobei der Tupfer (5, 5', 5") ein Desinfektionsmittel aufweist.

8. Schutzkappe (1 , 1') nach einem der vorhergehenden Ansprüche, wobei der äußere Kappenbereich (2) ein Befestigungsmittel aufweist zum Befestigen der Schutzkappe (1, 1') an dem Konnektor (20).

9. Schutzkappe (1, 1') nach Anspruch 8, wobei das Befestigungsmittel zum lösbaren Befestigen der Schutzkappe (1 , 1') an dem Konnektor (20) ausgebildet ist, beispielsweise der äußere Kappenbereich (2) ein Innengewinde (8) aufweist, oder der äußere Kappenbereich (2) ein oder mehrere federnd ausgebildete Rastnasen aufweist zum lösbaren Hintergreifen von ein oder mehreren Hinterschnitten (24) am Konnektor (20).

10. Schutzkappe (1, 1') nach Anspruch 8, wobei das Befestigungsmittel zum nichtlösbaren Befestigen der Schutzkappe (1, 1') an dem Konnektor (20) ausgebildet ist, beispielsweise der äußere Kappenbereich (2) ein oder mehrere federnd ausgebildete Rastnasen (8') aufweist zum dauerhaften Hintergreifen von ein oder mehreren Hinterschnitten (24) am Konnektor (20).

11. Schutzkappe (1 , 1') nach einem der vorhergehenden Ansprüche, zusätzlich umfassend eine Sperrfolie (9), die den den Tupfer (5, 5', 5") umfassenden Innenbereich (3) der Schutzkappe (1, 1') luftdicht verschließt.

12. Konnektorsystem, umfassend eine Schutzkappe (1 , 1') nach einem der Ansprüche 1 bis 11 und einen Konnektor (20), wobei der Konnektor (20) ein Anschlussstück (21) und eine zum Rand (22) des Anschlussstücks (21) zurückgesetzte Membran (23) aufweist, wobei Konnektor (20) und Schutzkappe (1 , 1') derart ausgebildet sind, dass bei auf dem Anschlussstück (11) des Konnektors (20) aufgesetzter Schutzkappe (1, 1') der Tupfer (5, 5', 5") der Schutzkappe (1, 1') die Membran (23) des Konnektors (20) berührt.

13. Konnektorsystem nach Anspruch 12, wobei Konnektor (20) und Schutzkappe (1, 1') derart ausgebildet sind, dass bei auf dem Anschlussstück (21) des Konnektors (20) vollständig aufgesetzter Schutzkappe (1, 1') der Tupfer (5, 5', 5") der Schutzkappe (1 , 1') die Membran (23) des Konnektors (20) berührt, aber nicht öffnet.

14. Konnektorsystem nach einem der Ansprüche 12 oder 13, wobei der Konnektor (20) einen Anschlag (24) aufweist, der eine Endstellung der Schutzkappe (1, 1') auf dem Konnektor (20) definiert.

15. Verfahren zum Reinigen und/oder Desinfizieren einer Oberfläche einer zurückgesetzten Membran (23) eines Konnektors (20) mit einer Schutzkappe (1, 1') nach einem der Ansprüche 1 bis 11, wobei die Schutzkappe (1, 1') auf ein Anschlussstück (21) des Konnektors (20) aufgesetzt und befestigt wird, und beim Erreichen einer Endstellung, in der die Schutzkappe (1, 1') am Konnektor (20) befestigt ist, der Tupfer (5, 5', 5") die Membran (23) berührt und reinigt und/oder desinfiziert.
